Europäisches Patentamt

**(19)** European Patent Office

Office européen des brevets

**(11)** Numéro de publication : **0 442 831 A1**

## **(12)** DEMANDE DE BREVET EUROPEEN

**(21)** Numéro de dépôt : **91420048.0**

**(22)** Date de dépôt : **11.02.91**

**(51)** Int. Cl.⁵ : **C08J 3/03, C08L 83/04, B01F 3/08, B01F 17/00, A61K 7/48**

**(30)** Priorité : **14.02.90 FR 9001990**

**(43)** Date de publication de la demande :
**21.08.91 Bulletin 91/34**

**(84)** Etats contractants désignés :
**AT BE CH DE DK ES FR GB GR IT LI LU NL SE**

**(71)** Demandeur : **RHONE-POULENC CHIMIE**
**25, quai Paul Doumer**
**F-92408 Courbevoie Cédex (FR)**

**(72)** Inventeur : **Bibette, Jérôme**
**158, cours du Général de Gaulle**
**F-33170 Gradignan (FR)**

**(74)** Mandataire : **Trolliet, Maurice et al**
**RHONE-POULENC CHIMIE, Direction des**
**Brevets, Centre de Recherches des Carrières,**
**BP 62**
**F-69192 Saint-Fons Cédex (FR)**

**(54)** Procédé de préparation d'émulsions, du type huile dans eau, monodisperses.

**(57)** La présente invention concerne un procédé de préparation d'émulsions monodisperses, à partir d'une émulsion polydisperse stable au stockage, du type "huile" dans eau, préparée à partir d'"huile", d'eau et d'un tensio-actif, caractérisé en ce que :

a) - on ajuste la teneur en huile de l'émulsion à une valeur comprise entre 1 et 40 % en poids,

b) - on augmente la concentration en tensio-actif de l'émulsion polydisperse par addition d'une quantité de tensio-actif efficace pour obtenir deux phases, une phase dite liquide où les gouttelettes d'"huile" sont libres et une phase dite solide où les gouttelettes sont associées,

c) - on sépare la phase liquide de la phase solide, et

d) - on recommence éventuellement les opérations a), b) et c) sur la phase solide ainsi séparée autant de fois qu'il est nécessaire pour obtenir une émulsion monodispersée présentant la granulométrie désirée.

Application à la préparation d'émulsions monodispersées huile dans eau, l'huile étant en particulier un polydiorganosiloxane.

# PROCEDE DE PREPARATION D'EMULSIONS, DU TYPE HUILE DANS EAU, MONODISPERSES

La présente invention concerne un procédé de préparation d'émulsions, du type huile dans eau, monodisperses ou à distribution granulométrique ressérée.

Les émulsions ioniques ou non ioniques, du type huile dans eau, sont des systèmes colloïdaux, obtenus à partir de trois composants de base, l'eau, l'"huile" et le tensio-actif. Dans ces systèmes colloïdaux, une phase se trouve dispersée artificiellement dans une autre.

Les émulsions huile dans eau sont donc formées d'une phase essentiellement constituée de gouttelettes d'"huile" dispersées dans une phase continue aqueuse.

Ces gouttelettes d'huiles sont entourées d'un film de tensio-actif, elles constituent les particules de l'émulsion dont la granulométrie peut varier de quelques centaines d'Angstroem environ à plusieurs dizaines de $\mu$m.

La distribution granulométrique des gouttelettes d'"huile" obtenues selon les procédés classiques est généralement étalée. Les émulsions correspondantes seront également dénommées émulsions polydispersées.

Pour une distribution du type de Gauss ayant un diamètre médian de 1 $\mu$m, on considérera cette distribution comme étalée (émulsion polydispersée) si l'écart type relatif est de l'ordre de 60 %, ce qui signifie qu'environ les 2/3 en poids des gouttelettes ont un diamètre compris entre 0,4 et 1,6 $\mu$m.

On considérera comme resserrée toute distribution (émulsion monodispersée) dont l'écart type est inférieur ou égal à 30 %, et de préférence de l'ordre de 15 à 25 %.

Le but de l'invention est donc d'obtenir des distributions resserrées de gouttelettes (écart type inférieur ou égal à 30 %, de préférence de l'ordre de 15 à 25 %, voir même de l'ordre de 3 à 5 % en augmentant le nombre de fractionnements). Les émulsions correspondantes seront donc également dénommées émulsions monodispersées.

Dans le cas contraire, elle est dite polydisperse.

Les émulsions du type ci-dessus, sont dans la plupart des cas obtenues sous une forme très polydisperse par les procédés conventionnels.

Certains appareillages spéciaux et coûteux permettent cependant d'aboutir à des émulsions monodisperses mais dont la taille des particules est énorme (100 $\mu$ d'après l'exemple de EP-A-328 118).

Toutefois les émulsions monodisperses possèdent des propriétés remarquables qui peuvent être mises à profit dans de nombreuses applications, notamment en pharmacologie, optique mécanique et lubrification et, l'objet de la présente invention est précisément de proposer un procédé simple, efficace et peu coûteux, de préparation d'émulsions monodisperses à partir d'émulsions polydisperses.

La présente invention concerne en effet un procédé de préparation d'émulsions monodisperses, à partir d'une émulsion polydisperse stable au stockage, du type "huile" dans eau, préparée à partir d'"huile", d'eau et d'un tensio-actif, caractérisé en ce que :

a) - on ajuste la teneur en huile de l'émulsion à une valeur comprise entre 1 et 40 % en poids,

b) - on augmente la concentration en tensio-actif de l'émulsion polydispersée par addition d'une quantité de tensio-actif efficace pour obtenir deux phases, une phase dite liquide où les gouttelettes d'"huile" sont libres et une phase dite solide où les gouttelettes sont associées,

c) - on sépare la phase liquide de la phase solide,

d) - on recommence éventuellement les étapes a), b) et c) sur la phase solide ainsi séparée autant de fois qu'il est nécessaire pour obtenir une émulsion monodisperse présentant la granulométrie désirée,

e) - on récupère l'émulsion monodispersée que l'on dilue éventuellement afin d'obtenir la teneur en extrait sec désirée.

Par "huile" on entend selon l'invention toute substance hydrophobe, insoluble ou très peu soluble dans l'eau, susceptible d'être mise en émulsion aqueuse, stable du type huile dans eau, à l'aide d'au moins un tensio-actif. Une telle substance hydrophobe et insoluble peut être par exemple un polymère organique, un latex organique et un polyorganosiloxane qui peuvent être mis sous forme d'émulsions polydisperses du type huile dans eau par des méthodes chimiques et/ou mécaniques en présence d'un tensio-actif.

Ces émulsions polydisperses, ioniques ou non ioniques, doivent être en outre stables au stockage, c'est-à-dire résister à la coalescence des gouttelettes d'huile de sorte qu'elles demeurent dans l'état qui a suivi leur préparation.

Le procédé selon l'invention est plus particulièrement performant dans le cas où l'"huile" est un polymère organosilicié polyorganosiloxane, c'est-à-dire une silicone.

On sait mettre en émulsions aqueuses pratiquement toutes les silicones qui se présentent sous forme de polymères fluides plus ou moins visqueux, ou solides à température ambiante.

Les polymères silicones sont linéaires, cycliques ou ramifiés. Les polymères très ramifiés sont également dénommés résines. Ils peuvent se présenter sous forme de fluides plus ou moins visqueux ou de solides.

Les polydiorganosiloxanes sont des polymères sensiblement linéaires qui se présentent sous forme de fluides plus ou moins visqueux allant des huiles peu visqueuses jusqu'aux gommes.

Pour préparer des émulsions silicones plusieurs procédés sont utilisables. Ces procédés aboutissent généralement à des émulsions polydisperses du type huile dans eau.

Selon un premier procédé, on mélange le polyorganosiloxane avec l'agent tensio-actif, ce dernier pouvant être déjà en solution aqueuse, puis on ajoute ensuite, si nécessaire, de l'eau. On transforme ensuite l'ensemble en une émulsion fine et homogène par passage dans un broyeur classique à colloïdes. Ce premier procédé est mis en oeuvre par exemple dans les brevets français FR-A-2 064 563, FR-A-2 094 322, FR-A-2 114 230 et FR-A-2 114 230 et le brevet européen EP-A-169 098.

Selon une variante de ce procédé, on prémélange l'agent tensio-actif et au moins une partie de l'eau à une température comprise entre la température ambiante (25 °C) et 80 °C jusqu'à l'obtention d'un mélange homogène, puis on ajoute lentement le polyorganosiloxane au prémélange, tout en agitant énergiquement à une température comprise entre 25 et 80 °C. On règle la consistance de l'émulsion homogène obtenue en ajoutant éventuellement de l'eau. Ce deuxième procédé est par exemple décrit dans les brevets français FR-A-2 471 210 et FR-A-2 485 923.

Des procédés de préparation d'émulsions utilisables comme produit de départ pour la mise en oeuvre du procédé selon l'invention et plus spécifiquement adaptés à la mise en émulsion de résines silicones, sont par exemple décrits dans les brevets américains US-A-4 028 339, US-A-4 052 331, US-A-4 056 492, US-A-4 525 502 et US-A-4 717 599 cités comme références.

Un deuxième procédé applicable aux polymères linéaires ou ramifiés consiste à polymériser en émulsion l'organopolysiloxane. Ce procédé est décrit en détail dans les brevets américains US-A-2 891 920, US-A-3 294 725, US-A-3 360 491 et les brevets européens EP-A-266 729 et EP-A-304 719.

Selon ce procédé le catalyseur de polymérisation peut être soit différent du tensio-actif, soit jouer également le rôle de tensio-actif.

Les émulsions polydisperses de départ contiennent un agent tensio-actif, de préférence non ionique ou anionique, mais on peut également utiliser des tensio-actifs cationiques et amphotères.

Ces tensio-actifs sont bien connus de l'homme de métier et sont en particulier décrits dans les brevets américains US-A-2 891 920, US-A-3 294 725, US-A-3 360 491, US-A-3 983 148 et le brevet français FR-A-2 605 634 cités comme référence.

Les agents tensio-actifs anioniques sont choisis parmi les alkylbenzènesulfonates de métaux alcalins, alkylsulfates de métaux alcalins tel que le dodécylsulfate de sodium, les alkyléthersulfates de métaux alcalins, les alkylaryléthersulfates de métaux alcalins et les dioctylsulfosuccinates de métaux alcalins.

Les agents tensio-actifs cationiques utilisés conformément à l'invention, sont choisis parmi les halogénures de dialkyl($C_{10}$-$C_{30}$)benzyldiméthylammonium et les sels d'ammonium quaternaires polyéthoxylés.

Les agents tensio-actifs amphotères utilisés conformément à l'invention sont choisis parmi les N-alkyl($C_{10}$-$C_{22}$)bétaïnes, les N-alkyl($C_{10}$-$C_{22}$)amidobétaïnes, les alkyl($C_{10}$-$C_{22}$)imidazolines et les dérivés de l'asparagine.

Les agents tensio-actifs non ioniques sont choisis parmi les acides gras polyéthoxylés, les esters de sorbitan, les esters de sorbitan polyéthoxylés, les alkylphénols polyéthoxylés, les alcools gras polyéthoxylés, les amides gras polyéthoxyles ou polyglycérolés, les alcools et alphadiols polyglycérolés.

Le procédé selon l'invention est avantageusement applicable aux émulsions silicones dont la silicone est un polydiorganosiloxane sensiblement linéaire bloqué à chacune de ses extrêmités par une fonction silanol ou par un radical triorganosiloxyle et dont la viscosité à 25 °C est généralement comprise entre 25 mPa.s et 30 x $10^6$ mPa.s.

Le polydiorganosiloxane peut donc être indifféremment une huile, une huile visqueuse et une gomme. Les radicaux organiques liés au silicium sont des radicaux hydrocarbonés monovalents généralement choisis parmi les radicaux alkyle en $C_1$-$C_{15}$, phényle, vinyle et l'atome d'hydrogène.

Les émulsions polydisperses de ces polydiorganosiloxanes ont une taille de gouttelettes comprise entre 0,15 et quelques dizaines de μm, le plus souvent entre 0,3 μm et quelques μm.

Selon l'étape a) du procédé de l'invention on ajuste la quantité d'eau de l'émulsion polydispersée de départ de façon à ce que la teneur pondérale en polydiorganosiloxane soit comprise entre 1 et 40 % en poids, de préférence, entre 4 et 15 % et, au cours de l'étape b), on augmente la concentration en tensio-actif de l'émulsion polydispersée par addition d'une quantité de tensio-actif efficace pour obtenir deux phases, une phase dite liquide où les gouttelettes d'huile sont libres et une phase dite solide où les gouttelettes sont associées.

La concentration initiale du tensio-actif à l'étape a) peut varier dans des proportions importantes, elle dépend en particulier de la quantité d'eau présente dans l'émulsion et de la nature du tensio-actif. Généralement elle au moins égale à la concentration micellaire critique du tensio-actif utilisé. De préférence elle est située entre 1 et 2 fois la (CMC). La CMC est la concentration en tensio-actif nécessaire à l'apparition des premiers aggrégats de tensio-actifs appelés mycelles et on dit que l'émulsion de l'huile dans l'eau n'est possible que si cette concentration en tensio-actif est au moins égale à la CMC.

Bien entendu l'émulsion de l'étape a) est toujours polydisperse et monophasique.

On entend selon l'invention par émulsion monophasique, une émulsion qui ne manifeste pas de décantation liée à la formation d'une phase dite solide.

Au cours de l'étape b) on augmente la concentration en tensio-actif par addition d'une quantité efficace de tensio-actif, c'est-à-dire la quantité nécessaire pour que l'émulsion de départ se sépare en deux phases, une phase liquide où les gouttelettes sont libres et une phase solide où les gouttelettes sont associées en aggrégats. Dans le cas où l'huile présente une densité plus faible que l'eau, la phase solide en équilibre avec la phase liquide décante vers le haut.

Pour obtenir une telle décantation, il suffit généralement de rajouter au cours de l'étape b) entre 0,5 et 10 fois, généralement entre 1 et 4 fois la concentration micellaire critique (CMC) de tensio-actif.

Après séparation complète des deux phases, qui peut être accélérée par une légère centrifugation, on sépare la phase liquide de la phase solide au cours de l'étape c). Cette séparation peut se faire par tout moyen, par exemple par écrèmage, par pompage et par aspiration.

Puis on recommence éventuellement les opérations a), b) et c) sur la phase solide autant de fois qu'il est nécessaire pour obtenir une émulsion monodisperse présentant la granulométrie désirée, à partir de la phase solide séparée.

Cette phase solide est éventuellement homogénéisée et diluée à l'eau pour obtenir une émulsion monophasée présentant une teneur en extrait sec désirée et où les gouttelettes d'huile sont bien séparées. La ou les phases solides ainsi séparées par le procédé de l'invention sont de plus en plus monodisperses. La taille des particules de la phase solide est mesurée de préférence par microscopie optique.

Bien entendu la phase liquide récupérée en c), peut être considérée comme une émulsion de départ de l'étape a) mais dépourvue de la population de particules récupérées dans la phase solide. Par conséquent on peut sur cette phase liquide appliquer au moins une fois les étapes b) et c) du procédé de l'invention.

Les phases solides générées par des concentrations en tensio-actif de plus en plus grandes seront constituées de particules de plus en plus petites. Ceci étant une conséquence de l'action du tensio-actif et constitue le fondement du procédé selon l'invention.

Dans le cas des émulsions silicones on aboutit au résultat visé en répètant de 1 à 8 fois, généralement de 2 à 5 fois les étapes a), b) et c).

Bien entendu plus on répète les trois étapes a), b) et c) plus on engendre un fractionnement de l'émulsion initiale, ce résultat étant analogue à une procédé de cristallisation fractionnée.

On peut également ajouter au cours de l'étape b) un tensio-actif différent de celui présent à l'étape a). Néanmoins il est souhaitable de mettre en oeuvre les étapes a) et b) avec le même tensio-actif.

Les exemples suivants illustrent l'invention.

Dans ce qui suit ou ce qui précède, sauf mentions contraires, les pourcentages et parties sont en poids.

- EXEMPLE 1 -

On mélange vigoureusement pendant 15 minutes :
– 400 parties d'huile polydiméthylsiloxane bloquée à chacune de ses extrémités par un motif triméthylsiloxyle de viscosité 100 mPa.s à 25 °C,
– 50 parties de laurylsulfate de sodium.

Tout en maintenant l'agitation on coule pendant 15 minutes supplémentaires, 60 parties d'eau.

On passe le mélange dans un broyeur à colloïde MORITZ® et on dilue le broyat obtenu avec 400 parties d'eau. L'émulsion est mélangée pendant une heure, puis filtrée.

On ajoute alors la quantité nécessaire d'eau, de façon à ce que la concentration en poids d'huile silicone dans l'émulsion polydispersée soit de 10 % en poids.

La concentration $C_i$ de tensio-actif est alors de 0,02 mole/l.

Les gouttelettes présentent une granulométrie initiale comprise entre 0,3 et 5 µm.

On ajoute alors à l'émulsion du même tensio-actif de façon à ce que sa concentration soit portée à la concentration $CO = 0,04$ mole/l.

On laisse reposer l'émulsion 12 heures. Après 12 heures, deux phases apparaissent, la phase inférieure $Lo$ étant la phase dite liquide où les gouttelettes sont distinctes, et la phase $So$ étant dite phase solide où les gouttelettes sont agglomérées.

Cette phase $So$, contenant les gouttelettes (particules) agglomérées est prélevée par aspiration.

On porte alors la concentration en tensio-actif de la phase liquide $Lo$ ainsi recueillie à la concentration $C_i = 0,045$ mole/l, on laisse reposer 12 heures.

L'émulsion se sépare en deux phases, la phase inférieure $L1$ étant la phase dite liquide, la phase supérieure S1 étant dite phase solide contenant les gouttelettes agglomérées qui est prélevée par aspiration.

On répète les opérations en portant la concentration en tensio-actif dans L1 à la valeur C2 de 0,05 mole/l et on répète jusqu'à L6, les résultats obtenus sont assemblés dans le tableau 1 ci-après où d0 à d6 représentent la taille moyenne des gouttelettes présentes dans les phases S0 à S6 et t représente l'écart type relatif en % de la distribution de taille des gouttelettes.

- EXEMPLE 2 : on part de la phase S2 obtenue à l'exemple 1.

Cette phase S2 est redispersée sous agitation dans l'eau pure, de telle sorte que la teneur pondérale dans l'huile soit de 10 % en poids. La concentration initiale en tensio-actif C2i est de 0,01 mole/l. On ajoute du tensio-actif pour que la concentration en tensio-actif soit C20 = 0,045 mole/litre. On sépare la phase liquide L20 de la phase solide S20.

On répète 2 fois les opérations en augmentant la concentration en tensio-actif afin de recueillir les solides S21 et S22.

Les résultats obtenus sont rassemblés dans le tableau 2 ci-après où d20, d21, d22 représentent la taille moyenne des gouttelettes présentes dans les phases S20, S21 et S22 et t représente l'écart type relatif en %.

## TABLEAU 2

| C   | mole/l | S   | d (en $\mu$m) | t (%) |
|-----|--------|-----|---------------|-------|
| C20 | 0,045  | S20 | d20 = 0,8     | 20 %  |
| C21 | 0,050  | S21 | d21 = 0,7     | 20 %  |
| C22 | 0,055  | S22 | d22 = 0,6     | 20 %  |

Le volume des phases S20 et S22 est très faible en général.

La phase S21 est récupérée et diluée à 10 % et on porte la concentration en tensio-actif à une valeur de C220 de 0,048 mole/l.

On sépare une phase solide S220 et une phase liquide L220.

On répète deux fois les opérations en augmentant la concentration en tensio-actif afin de recueillir successivement les phases S21 etS22.

Les résultats obtenus sont rassemblés dans le tableau 3 ci-après. Le volume des phases S220 et S222 est très faible. Dans le tableau 3 d220, d221, d222 représentent la granulométrie en nombre des gouttelettes présentes dans les phases S220, S221, S222 et t représente l'écart type relatif en %.

TABLEAU 3

| C | mole/l | S | d (en μm) | t (%) |
|---|---|---|---|---|
| C220 | 0,048 | S220 | d220 = 0,7 | 15 % |
| C221 | 0,050 | S221 | d221 = 0,65 | 15 % |
| C222 | 0,052 | S222 | d222 = 0,6 | 15 % |

TABLEAU 1

| C | mole/l | S | d (en μ) | t (%) |
|---|---|---|---|---|
| Co | 0,04 | S0 | 1 < do < 5 | 30 |
| C1 | 0,045 | S1 | 0,8 < d1 < 1 | 30 |
| C2 | 0,050 | S2 | 0,6 < d2 < 0,8 | 30 |
| C3 | 0,055 | S3 | 0,5 < d3 < 0,6 | 30 |
| C4 | 0,060 | S4 | 0,4 < d4 < 0,5 | 30 |
| C5 | 0,065 | S5 | 0,35 < d5 < 0,40 | 30 |
| C6 | 0,070 | S6 | d6 < 0,35 | 30 |

**Revendications**

**1.** - Procédé de préparation d'émulsions monodisperses, à partir d'une émulsion polydisperse stable au stockage, du type "huile" dans eau, préparée à partir d'"huile", d'eau et d'un tensio-actif, caractérisé en ce que:

a) - on ajuste la teneur en huile de l'émulsion à une valeur comprise entre 1 et 40 % en poids,

b) - on augmente la concentration en tensio-actif de l'émulsion polydisperse par addition d'une quantité de tensio-actif efficace pour obtenir deux phases, une phase dite liquide où les gouttelettes d'"huile" sont libres et une phase dite solide où les gouttelettes sont associées,

c) - on sépare la phase liquide de la phase solide,

d) - on recommence éventuellement les étapes a), b) et c) sur la phase solide ainsi séparée autant de fois qu'il est nécessaire pour obtenir une émulsion monodisperse présentant la granulométrie désirée,

e) - on récupère l'émulsion monodispersée que l'on dilue éventuellement afin d'obtenir la teneur en extrait sec désirée.

**2.** - Procédé selon la revendication 1, caractérisé en ce que la concentration en tensio-actif de l'émulsion a) est au moins égale à la concentration micellaire critique.

**3.** - Procédé selon la revendication 1, caractérisé en ce que l'"huile" est choisie parmi un polymère organique, un latex et un polyorganosiloxane.

**4.** - Procédé selon la revendication 1, 2 ou 3, caractérisé en ce que l'"huile" est un polydiorganosiloxane sensiblement linéaire bloqué à chacune de ses extrémités par une fonction silanol ou par un radical triorganosiloxyle et dont la viscosité à 25 °C est comprise entre 25 mPa.s et $30 \times 10^6$ mPa.s.

**5.** - Procédé selon la revendication 4, caracterisé en ce que le tensio-actif est chosi parmi les tensio-actifs anioniques et non-ioniques.

**6.** - Procédé selon la revendication 5, caractérisé en ce que le tensio-actif est un laurylsulfate de métal alcalin.

**7.** - Procédé selon l'une quelconque des revendications 2 à 6, caractérisé en ce qu'on ajoute au cours de l'étape b) de 0,5 à 10 % fois la concentration micellaire critique de tensio-actif.

**8.** - Procédé selon l'une quelconque des revendications précédentes, caractérisé en ce que sur la phase liquide récupérée en c), on applique encore au moins une fois les étapes b) et c).

Office européen
des brevets

**RAPPORT DE RECHERCHE EUROPEENNE**

Numéro de la demande

EP 91 42 0048

| **DOCUMENTS CONSIDERES COMME PERTINENTS** | | | |
|---|---|---|---|
| Catégorie | Citation du document avec indication, en cas de besoin, des parties pertinentes | Revendication concernée | CLASSEMENT DE LA DEMANDE (Int. Cl.5) |
| A | US-A-4 824 877 (S.O. GLOVER et al.) * revendications; exemple 1 * --- | 1,3-6 | C 08 J 3/03 C 08 L 83/04 B 01 F 3/08 B 01 F 17/00 A 61 K 7/48 |
| A | EP-A-0 138 192 (DOW CORNING CORPORATION) * revendications 1,2,9,10 * --- | 1,3-5 | |
| A | FR-A-2 624 867 (DOW CORNING LTD.) * revendication 1 * --- | 1,3-5 | |
| A | EP-A-0 228 575 (DOW CORNING CORPORATION) * revendication 1; exemple 2 * ----- | 1 | |

**DOMAINES TECHNIQUES RECHERCHES (Int. Cl.5)**

C 08 J
C 08 L
C 08 F
B 01 F
A 61 K

Le présent rapport a été établi pour toutes les revendications

| Lieu de la recherche | Date d'achèvement de la recherche | Examinateur |
|---|---|---|
| BERLIN | 24-04-1991 | CORDERO ALVAREZ M. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

EPO FORM 1503 03.82 (P0402)

8